# FASCICULE DE BREVET EUROPEEN

(11) **EP 4 319 635 B1**
(45) Date de publication et mention de la délivrance du brevet: **05.08.2026**
(21) Numéro de dépôt: 22719980.9
(22) Date de dépôt: 08.04.2022
(51) Int. Cl.: A61B 5/145, A61B 5/00, H01R 24/00, H01R 24/66, H01R 24/76, H01R 13/17

(54) **DISPOSITIF DE SURVEILLANCE CORPORELLE COMPRENANT DES CONTACTS ÉLECTRIQUES AMÉLIORÉS**
KÖRPERÜBERWACHUNGSVORRICHTUNG MIT VERBESSERTEN ELEKTRISCHEN KONTAKTEN
BODY MONITORING DEVICE WITH IMPROVED ELECTRICAL CONTACTS

(30) Priorité: 09.04.2021 FR 2103653
(43) Date de publication de la demande: 14.02.2024
(73) Titulaire: WIZP AS, 3125 Tønsberg (NO)
(72) Inventeur: PIERART, Luc, 94800 VILLEJUIF (FR); HUANG, Yulin, TAIPEI (TW); BADIOLA, Florian, 75006 PARIS (FR)
(74) Mandataire: AWA Norway AS
(86) Numéro de dépôt international: PCT/FR2022/050658
(87) Numéro de publication internationale: WO 2022/214770

(56) Documents cités:
- EP-A1- 3 603 508
- WO-A1-2019/241753
- FR-A1- 3 076 704
- US-A1- 2008 288 026
- US-A1- 2010 049 021
- US-A1- 2017 202 512
- JASON: "6Pin Female&Male Pogo pin connector", 30 October 2017 (2017-10-30), XP055865419, Retrieved from the Internet <URL:https://www.pogo-pins.com/products/642.html> [retrieved on 20211124]
- ANONYMOUS: "Why use a concave vs. flat mating target for pogo pins? - Electrical Engineering Stack Exchange", 20 September 2019 (2019-09-20), XP055941952, Retrieved from the Internet <URL:https://electronics.stackexchange.com/questions/459496/why-use-a-concave-vs-flat-mating-target-for-pogo-pins> [retrieved on 20220713]

## Description

### DOMAINE TECHNIQUE

L'invention concerne un dispositif de surveillance corporelle et en particulier un dispositif de surveillance corporelle par une analyse de liquide corporel, typiquement interstitiel au moyen de microaiguille(s).

### ETAT DE LA TECHNIQUE

La surveillance de nombreuses maladies chroniques connues chez l'humain nécessite un relevé quotidien de paramètres biochimiques. Un niveau de concentration d'un analyte corporel dans un fluide corporel de l'organisme, par exemple dans le plasma sanguin ou dans le liquide interstitiel des cellules de l'organisme, peut être relevé.

A titre d'exemple répandu, le suivi du diabète chez un patient nécessite un relevé précis quotidien de la glycémie du patient.

Une solution répandue pour réaliser le suivi du diabète consiste à réaliser une ponction, par exemple au bout du doigt, pour faire perler une goutte de sang, puis à réaliser une mesure quotidienne de glycémie dans la goutte de sang ainsi obtenue.

Des systèmes de suivi ont été proposés pour se passer de la nécessité d'une ponction manuelle, de sorte à rendre la mesure de glycémie moins laborieuse et moins invasive. On parle de systèmes CGM, pour « Continuous Glucose Monitoring ». Certains de ces systèmes CGM réalisent, à intervalles réguliers, une mesure de glycémie au niveau du liquide interstitiel entre les cellules de la peau. La glycémie du liquide interstitiel est très proche de la glycémie du plasma sanguin. Les mesures au niveau du liquide interstitiel permettent un suivi simple et peu invasif de la glycémie des patients ; ces mesures peuvent être réalisées à l'aide de capteurs à aiguille, en transcutané, ou de manière non invasive, comme par exemple en iontophorèse ou en implantable avec une mesure par chimio-fluorescence.

La demande internationale publiée sous le numéro WO 2018/104647 décrit un système de surveillance corporelle, utilisable notamment pour le suivi de la glycémie. Ce système de surveillance inclut une montre électronique attachable au poignet à l'aide d'un bracelet. La montre comporte un boîtier, dans lequel s'insère une capsule amovible interchangeable comprenant un capteur à micro-aiguilles. Le capteur est commandé de manière automatique par l'électronique du boîtier, pour réaliser une mesure transcutanée. La mesure de glycémie par le capteur est une mesure électrochimique.

Le dispositif comprend des connecteurs qui permettent de connecter électriquement le capteur au boitier, en particulier chaque connecteur comprend un partie male disposée sur le boitier et une partie femelle disposée sur le capteur.

Toutefois, lorsqu'un tel dispositif est porté au poignet, la connexion entre le capteur et le boitier peut être fluctuante compte tenu des différences d'enfoncements des aiguilles dans la peau ou compte tenu des mouvements du poignet de l'utilisateur. Ainsi, la connexion entre le capteur et le boitier peut être imparfaite ce qui peut nuire aux mesures et donc à la surveillance.

L'état de la technique pertinent peut être trouvé dans les publications de brevets EP 3 603 508 A1, FR 3 076 704 A1 et US 2010/049021 A1.

### EXPOSE DE L'INVENTION

L'invention est définie dans l'ensemble de revendications ci-joint.

### EXPOSE DE LA DIVULGATION

La divulgation propose de pallier au moins un de ces inconvénients et propose à cet effet, un dispositif de surveillance corporelle, comprenant :
- un boitier comprenant une face inférieure ;
- un capteur comprenant une face supérieure agencée en face de la face inférieure du boitier, le boitier étant relié électriquement au capteur par l'intermédiaire d'au moins un connecteur, chaque connecteur comprenant une partie femelle formée dans l'épaisseur du capteur et une partie male en saillie de la face inférieure du boitier, la partie femelle comprenant un orifice de réception concave, ledit orifice étant tel que lorsque la partie male est insérée dans l'orifice la surface de contact possible entre la partie male et l'orifice est maximisée en cas déplacement relatif entre la partie male et la partie femelle.

La divulgation est avantageusement complétée par les caractéristiques suivantes, prises seules ou en une quelconque de leur combinaison techniquement possible :
- l'orifice est sphérique, ou conique ou tronconique ;
- l'orifice présente un angle d'ouverture compris entre 100°, et 160°, de préférence 130° ;
- la partie male est une broche en saillie de la face inférieure du boitier ;
- la broche comprend une partie fixe attachée au boitier et une partie mobile par rapport à la partie fixe, la broche étant configurée pour permettre un déplacement de la partie mobile par rapport à la partie fixe selon une direction de translation perpendiculaire à la face supérieure du capteur ;
- la broche comprend un élément élastique attaché à la partie mobile et à la partie fixe de la broche, l'élément élastique étant de préférence un ressort ;
- la partie male est constituée d'une lame ressort s'étendant depuis la face interne du boitier et est conformée pour présenter une partie configurée pour être introduite dans l'orifice ;
- la partie femelle est en outre configurée pour être connectée à un connecteur électrique disposé sur la partie inférieure du capteur ;
- la partie femelle est constituée par une pièce métallique disposée dans toute l'épaisseur du capteur, la pièce métallique comportant une partie concave au-dessus d'un support métallique conducteur ;
- la partie femelle comprend en outre une piste conductrice s'étendant depuis l'orifice vers la partie inférieure capteur en cheminant sur la surface externe du capteur ;
- la partie femelle comprend un via qui s'étend depuis l'orifice dans l'épaisseur du capteur ;
- le dispositif comprend au moins une microaiguille disposée sur la face inférieure du capteur.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre schématiquement un dispositif selon un mode de réalisation de l'invention en coupe ;
- la figure 2 illustre schématiquement un dispositif selon un mode de réalisation en vue éclatée selon un premier point de vue ;
- la figure 3 illustre schématiquement un dispositif selon un mode de réalisation en vue éclatée selon un deuxième point de vue ;
- la figure 4a, la figure 4b et la figure 4c illustrent plusieurs configurations d'un orifice d'un capteur du dispositif de l'invention ;
- la figure 5a, la figure 5b et la figure 5c illustrent une comparaison entre un contact plan de type connu et un contact amélioré selon l'invention ;
- la figure 6 illustre une partie male en forme de broche d'un dispositif selon l'invention ;
- la figure 7 illustre une partie male en forme de lame ressort d'un dispositif selon l'invention ;
- la figure 8a, la figure 8b et la figure 8c illustrent différentes possibilités pour connecter les faces inférieure et supérieure d'un capteur d'un dispositif selon la divulgation. La figure 8b illustre la connection des faces inférieure et supérieure d'un capteur d'un dispositif selon l'invention ;
- la figure 9 et la figure 10 illustrent l'enfoncement des parties males dans des orifices d'un dispositif selon l'invention.

Sur l'ensemble des figures les éléments similaires portent des références identiques.

### DESCRIPTION DETAILLEE

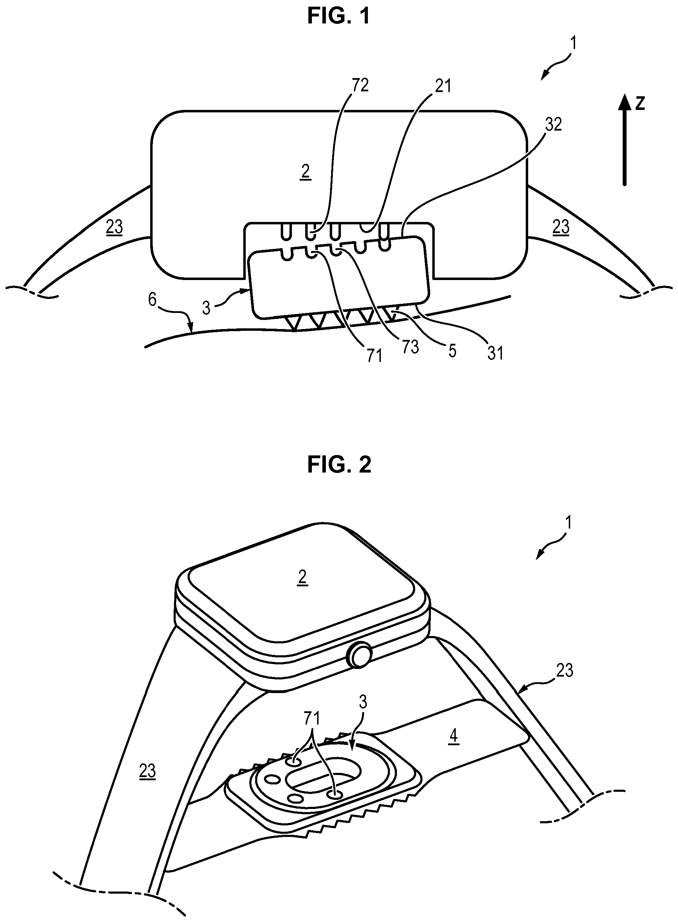

En relation avec les **figures 1, 2** et **3****,** un dispositif 1 de surveillance corporelle comprend un boitier 2, un capteur 3, un patch adhésif 4. Un tel dispositif 1 de surveillance est avantageusement utilisé pour mettre en oeuvre un procédé de surveillance corporelle.

Par surveillance corporelle, on entend la vérification de constantes biochimiques d'une personne porteuse du dispositif, typiquement la concentration en une protéine, une hormone, un marqueur, en oxygène, en nutriments, etc. dans un liquide corporel de la personne. A titre d'exemple on peut citer la glycémie. L'homme du métier pourra aussi surveiller si besoin d'autres grandeurs physiques corporelles telles que la température, l'hydratation, etc.

On considère également que la constante biochimique à surveiller est la concentration en glucose (ou glycémie) au sein du liquide interstitiel de la peau. La glycémie du liquide interstitiel est considérée comme représentative de la glycémie du plasma sanguin.

On considère ici que le liquide corporel est du liquide interstitiel mais on peut aussi considérer d'autres liquides corporels tels que le sang.

Le capteur 3 est un capteur à aiguilles prévu pour fournir une mesure de courant électrique au sein du liquide interstitiel du porteur du dispositif 1. Des aiguilles 5 sont avantageusement disposées sur une face interne 31 du capteur 3. Cette face interne 31 est destinée à être placée sur la peau 6 du porteur. Les aiguilles 5 sont avantageusement des micro-aiguilles. Le capteur 3 comporte de préférence entre quatre et cinquante micro-aiguilles voire quatre cent microaiguilles. Bien entendu, un nombre de différent peut être considéré sans que cela ne limite la description de l'invention ici faite.

On entend par micro-aiguille, une aiguille présentant une hauteur faible de préférence entre 10 µm et 1000 µm, de préférence entre 0,3 mm et 0,8mm. En outre, les micro-aiguilles sont sensiblement pyramidales. La hauteur des micro-aiguilles est suffisamment faible pour éviter le contact avec un nerf du porteur lorsque le dispositif est porté.

Les micro-aiguilles 5 permettent de mesurer ou prélever le liquide corporel.

Les microaiguilles 5 sont creuses lorsqu'il s'agit de prélever du liquide soit pleine pour analyser directement le liquide. Lorsqu'il s'agit de prélever le liquide, les microaiguilles permettent l'extraction de liquide interstitiel du derme de façon indolore sans perlement de sang et l'envoie vers un capteur logé dans le boitier 2. Lorsqu'il s'agit d'analyser du liquide, les microaiguilles ne prélèvent pas de liquide et intègrent le capteur sur leur surface sous la forme d'un revêtement tel qu'un matériau biochimique apte à réagir avec l'analyse à effectuer sur le liquide.

De manière avantageuse, le capteur 3 comprend plusieurs microaiguilles qui consistent en un réseau de microaiguilles en ce qu'elles sont électriquement connectées entre elles. Les microaiguilles transpercent la peau pour venir au contact du liquide interstitiel lorsque le capteur est au contact de la peau.

Comme illustré sur la figure 2, le capteur 3 est assemblé au patch adhésif 4 et peuvent ensemble constituer une capsule. Le capteur 3 peut aussi être amovible par rapport au patch 4. Une telle capsule est avantageusement montée amovible avec le boitier 2. En particulier, la capsule et donc le capteur 3 s'engage de manière préférée dans une cavité 21 du boitier 2 située sur sa face destinée à être en contact avec la peau. Le capteur 3 comprend en outre une face externe 32 opposée à la face interne 31.

Le capteur 3 ici illustré est de forme circulaire avec un orifice central 33 mais elle peut prendre d'autres formes : rectangulaire, oblongue, ellipsoïdale avec ou sans orifice central. L'orifice central 33 permet de positionner correctement le capteur 3 dans la cavité 21 du boitier qui comprend un plot central de positionnement (non représenté).

Le capteur 3 comprend donc des éléments qui permettent de prélever le liquide soit d'amener les signaux détectés par chaque microaiguille vers le boitier 2 pour traitement (non décrit ici).

Le capteur 3 peut prendre la forme d'une plaque de plastique, d'un circuit imprimé (rigide ou flexible en silicium), d'une plaque en métal non conducteur comme par exemple de l'aluminium.

Le patch adhésif 4 est adapté pour être collé à la peau et supporte le capteur 3 et permet de détacher le boitier 2 sans enlever le capteur 3 en la gardant collée au corps. Une telle configuration permet d'éviter d'enlever le capteur pour certaines opérations qui n'implique que le boitier : rechargement de la batterie, réparation, remplacement, extraction des données vers un ordinateur.

Le boitier 2 est avantageusement en forme de boitier de montre et comprend un bracelet 23 adapté pour entourer le poignet d'une personne. Le boitier 2 loge plusieurs éléments permettant d'analyser ou d'extraire du liquide interstitiel. A ce titre, on pourra se référer au document WO 2019/141743 au nom de la Demanderesse qui décrit en détail la mesure et la détection d'une grandeur physique à partir de microaiguilles au contact d'un liquide corporel pouvant être prélevé ou non.

Le contact électrique entre le capteur 3 et le boitier 2 est assuré par l'intermédiaire de plusieurs connecteurs 7. Ces connecteurs 7 permettent donc de connecter le capteur 3 au boitier 2. En effet, le boitier 2 doit pouvoir récupérer des informations issues du capteur 3 et doit pouvoir également l'alimenter.

Chaque connecteur 7 comprend une partie femelle 71 comprenant un orifice 73 formé dans l'épaisseur du capteur 2 et une partie male 72 en saillie d'une face inférieure 21 du boitier 2. On précise qu'une face inférieure sera plus proche du poignet qu'une face supérieure. L'orifice présente avantageusement une profondeur comprise entre 150 *µm* et 350 *µm,* de préférence 250 *µm* et le capteur 3 présente une épaisseur comprise entre 1 mm inclus et 3 mm inclus. Compte tenu de l'épaisseur du capteur 3 et qu'il doit être positionné autour du poignet d'un utilisateur, l'orifice 73 est nécessairement formé dans l'épaisseur du capteur 3.

L'orifice 73 est concave par rapport à la face supérieure 32 du capteur 3 depuis laquelle il est formé.

L'orifice 73 peut prendre plusieurs formes.

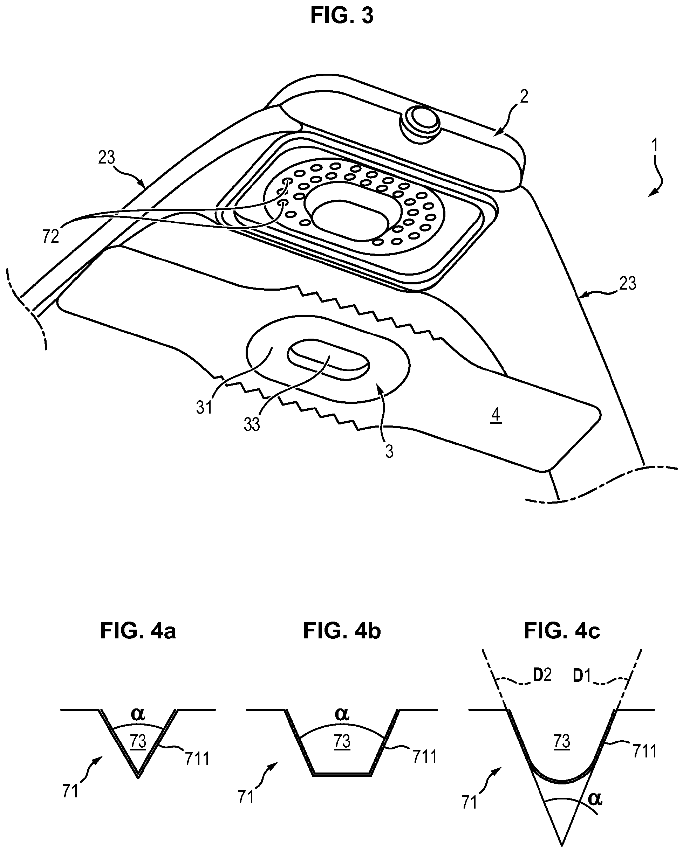

Selon un mode de réalisation illustré sur la **figure 4a****,** l'orifice 73 est conique.

Selon un mode de réalisation illustré sur la **figure 4b****,** l'orifice 73 est tronconique.

Selon un mode de réalisation illustré sur la **figure 4c****,** l'orifice 73 est sphérique

Quelle que soit la forme, l'orifice 73 présente un angle d'ouverture *α* compris entre 100 et 140 °C, de préférence égal à 130°.

Dans le cas de l'orifice 73 sphérique cet angle *α* est pris entre des tangentes D1, D2 à l'orifice 73 et qui partent des bords de l'orifice 73.

La partie femelle 71 comprend avantageusement un dépôt métallique conducteur 711 qui revêt l'intérieur de l'orifice 73. L'orifice 73 peut être formé sur toute ou partie de l'épaisseur du capteur 3. Chaque connecteur 7 est relié à une ou plusieurs microaiguille(s) et/ou à des pistes électriques et/ou à des électrodes.

L'orifice 73 de réception est donc conformé de manière à ce que lorsque la partie male 72 est insérée dans l'orifice 73 un contact électrique entre l'orifice 73 et la partie male 72 est maximisé afin d'optimiser le contact entre la partie male 72 et la partie femelle 71. En outre, ce contact est maximisé même en cas de déplacement de la partie male 72 dans l'orifice 73. En effet, la partie male 72 et la partie femelle 71 ne sont pas mécaniquement reliées entre elles, la forme donnée à l'orifice 73 compense cette absence de lien mécanique en compensant notamment les déplacements relatifs de la partie male 72 dans l'orifice 73.

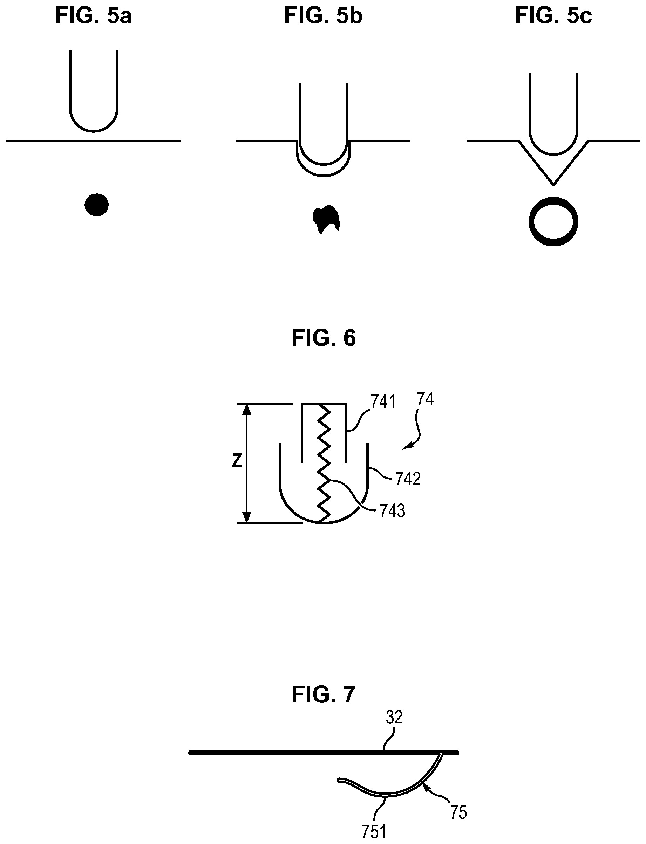

Comme visible sur les **figures 5a, 5b** et **5c****,** le fait de prévoir un orifice 73 concave autorise des mouvements de la partie male 71 dans l'orifice 73 sans que le contact électrique ne soit dégradé. En effet, entre un contact plan (voir la **figure 5a****)** et un contact avec l'orifice 73 concave, la surface de contact est augmentée (voir les **figures 5b** et **5c****).** Par exemple, la surface de contact (voir au-dessous de chacune de ces figures), la surface de contact avec l'orifice sphérique est supérieure à celle avec un contact plan, la surface de contact avec l'orifice conique est supérieure à celle avec l'orifice sphérique.

En effet, lorsque le dispositif est fixé au poignet d'un utilisateur et que le capteur 3 est attaché à la peau au moyen du patch adhésif, le boitier 2 vient se fixer au capteur 3 et est maintenu en position par le bracelet. Et, compte tenu de la position du dispositif autour du poignet on comprend que la pression exercée par le boitier sur le capteur n'est pas uniforme de sorte que les parties males ne s'enfoncent pas de la même manière dans les différents orifices. Ceci est aussi dû à l'enfoncement des micro-aiguilles qui n'est pas uniforme. En outre, les mouvements du dispositif peuvent faire bouger les parties males. Toutes ces raisons entrainent des mouvements des parties males par rapport à la parie femelle et sont compensés par la concavité de l'orifice puisque le contact électrique est maximisé. La connexion procurée par les connecteurs tels qu'ici décrits est donc robuste.

Selon un mode de réalisation, la partie male 71 est une broche en forme de cylindre métallique conducteur et présente une section sphérique. De cette manière elle peut parfaitement se loger dans l'orifice.

De manière préférée, comme illustré sur la **figure 6****,** la broche comprend une partie fixe 741 qui est attachée au boitier et une partie mobile 742 par rapport à la partie fixe 741, la broche 74 étant configurée pour permettre un déplacement de la partie mobile 742 par rapport à la partie fixe 741 selon une direction Z de translation perpendiculaire à la face inférieure 21 du boitier 2. La partie mobile 742 est coaxiale à la partie fixe 741. En outre, un élément élastique 743 tel qu'un ressort est fixé à l'intérieur de la broche entre la partie fixe 741 et la partie mobile. Un tel ressort est un ressort de compression de sorte que sans sollicitation de la broche selon la direction Z la partie mobile est plus éloignée de la partie fixe que sans sollicitation.

Selon une réalisation préférée, la partie fixe présente une hauteur de 2,5 mm et la partie mobile présente une hauteur de 0,6 mm. Toujours selon cette réalisation préférée, le ressort présente une raideur tell qu'il permet à la partie mobile de s'insérer dans la partie fixe d'environ 0,3 mm.

Selon un mode de réalisation illustré sur la **figure 7****,** la partie male 71 est constituée d'une lame métallique ressort s'étendant depuis la surface inférieure 21 du boitier 2. La lame métallique 75 est avantageusement conformée pour présenter une partie 751 configurée pour être introduite dans l'orifice 73. Cette partie 751 qui est destinée à être introduite dans l'orifice 73 est avantageusement sphérique. En outre, la lame 75 est en métal conducteur.

On comprend que la partie male du fait qu'elle comprend une partie élastique (broche ressort ou lame ressort) est configurée pour exercer une force sur le capteur de manière à compenser des variations quant à l'enfoncement des microaiguilles dans la peau ou aux mouvements du poignet du porteur. Il s'agit d'une compensation supplémentaire, la connexion n'en est que meilleure.

L'orifice présente une profondeur comprise entre 0,2 mm et 0,9 mm de préférence 0,5 mm. La profondeur est adaptée à la hauteur de la partie male qui doit venir se loger dans l'orifice.

De manière complémentaire, la partie femelle 71 est en outre configurée pour être connectée à un connecteur électrique 33 disposé sur la face inférieure 31 du capteur 3. Une telle connexion permet de relier électriquement les faces supérieure et inférieure notamment lorsqu'une micro-aiguille ou une électrode est connectée au connecteur électrique 33.

Pour connecter les faces supérieure et inférieure plusieurs modes de réalisation sont possibles.

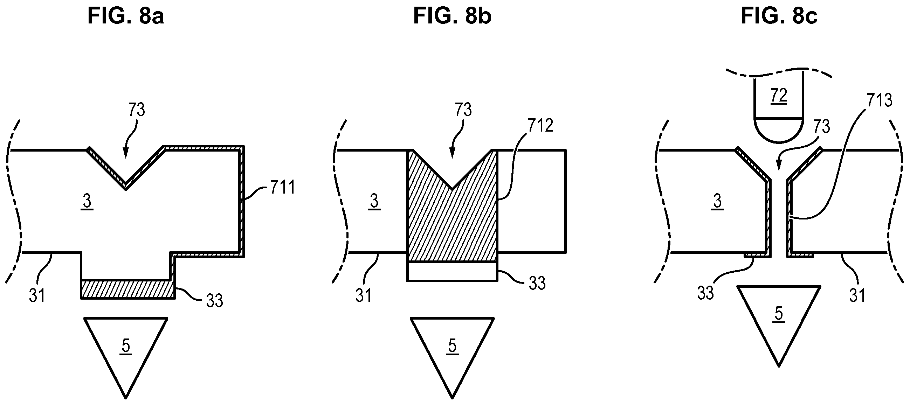

Selon un mode de réalisation (ne faisant pas partie de l'invention revendiquée) illustré sur la **figure 8a****,** la partie femelle 71 comprend une piste conductrice 711 s'étendant depuis l'orifice 73 vers la face inférieure 31 inférieure du capteur 3 en cheminant sur la surface externe du capteur 3. En particulier, la piste conductrice 711 chemine jusqu'au connecteur électrique 33 disposé sur la face inférieure du capteur 3.

Selon l'invention illustré sur la **figure 8b****,** la partie femelle 71 est constituée par une unique pièce métallique 712 disposée dans toute l'épaisseur du capteur 3, la pièce métallique 712 comportant une partie concave depuis laquelle s'étend un support métallique conducteur, cylindrique ou en forme de tige vers la surface inférieure du capteur 31.

Selon une mode de réalisation (ne faisant pas partie de l'invention revendiquée) illustré sur la **figure 8c****,** la partie femelle 71 comprend un via 713 qui s'étend depuis l'orifice dans toute l'épaisseur du capteur 3 jusqu'au connecteur électrique 33 disposé sur la face inférieure du capteur 3. Un métal conducteur revêt le via jusqu'au connecteur électrique 33.

En fonctionnement, lorsque le capteur 3 est amené vers le boitier 2 (ou l'inverse) afin de les connecter ensemble, les parties males du boitier doivent s'insérer dans les orifices. Compte tenu de la concavité de l'orifice et de leur angle d'ouverture, l'insertion des broches dans les orifices est simplifiée.

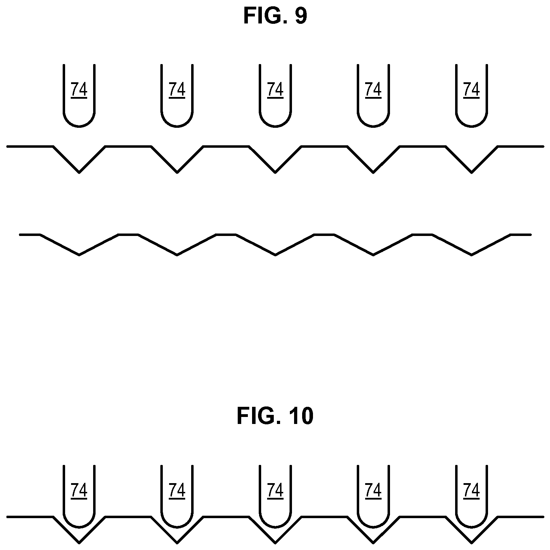

Ceci est illustré sur la **figure 9****,** plus l'angle d'ouverture des orifices est grand plus les erreurs d'alignement sont compensées. En effet, comme on peut la voir sur cette figure, on voit les broches 74 qui sont au-dessus d'orifice dont l'angle d'ouverture est de 45°. Des orifices avec une ouverture à 130° sont représentés au-dessous. On comprend immédiatement que l'insertion des broches sera possible dans des orifices présentant une ouverture large. Les phénomènes de blocage de l'insertion du fait d'un défaut d'alignement entre les parties males et les orifices sont donc atténués. Ceci est visible sur la **figure 10** où l'on voit que de tels orifices concaves avec une ouverture suffisamment large permettent d'assurer un contact électrique pour des insertions des parties males dans les orifices même si ces parties males sont insérées différemment dans les orifices.

## Revendications

1. Dispositif de surveillance corporelle, comprenant
- un boitier (2) comprenant une face inférieure (21) et un bracelet (23) adapté pour entourer le poignet d'un utilisateur ;
- un capteur (3) comprenant une face inférieure (31), une face supérieure (32) agencée en face de la face inférieure (21) du boitier (2) et au moins une microaiguille (5) disposée sur la face inférieure (31) du capteur (3),
- au moins un connecteur (7) reliant électriquement le boitier (2) au capteur (3), chaque connecteur (7) comprenant une partie femelle (71), la partie femelle (71) comprenant un orifice (73) de réception concave formé dans l'épaisseur du capteur (3) et une partie male (72) en saillie de la face inférieure (31) du boitier (2), l'orifice (73) étant concave par rapport à la face supérieure (32) du capteur (3) depuis laquelle l'orifice (73) est formé,
- un connecteur électrique (33) disposé sur la face inférieure (31) du capteur (3), une micro-aiguille étant connectée au connecteur électrique (33), la partie femelle (71) étant configurée pour être connectée au connecteur électrique (33) de manière à relier électriquement les face supérieure et inférieure du capteur (3),
l'orifice (73) étant tel que lorsque la partie male (72) est insérée dans l'orifice (73), la surface de contact entre la partie male et l'orifice est supérieure à celle avec un contact plan
dans lequel la partie femelle (71) est constituée par une pièce métallique (712) disposée dans toute l'épaisseur du capteur, la pièce métallique comportant la partie concave depuis laquelle s'étend un support métallique conducteur vers la surface inférieure (31) du capteur (3).
dans lequel le capteur (3) présente une épaisseur comprise entre 1 mm inclus et 3 mm inclus.

2. Dispositif selon la revendication 1, dans lequel l'orifice (73) est sphérique, ou conique ou tronconique.

3. Dispositif selon l'une des revendications 1 à 2, dans lequel l'orifice (73) présente un angle d'ouverture compris entre 100°, et 160°, de préférence 130°.

4. Dispositif selon l'une des revendications 1 à 3, dans lequel la partie male est une broche (74) en saillie de la face inférieure (31) du boitier.

5. Dispositif selon la revendication 4, dans lequel la broche comprend une partie fixe attachée au boitier (2) et une partie mobile par rapport à la partie fixe, la broche étant configurée pour permettre un déplacement de la partie mobile par rapport à la partie fixe selon une direction (Z) de translation perpendiculaire à la face supérieure (32) du capteur (3).

6. Dispositif selon la revendication 5, dans lequel la broche (74) comprend un élément élastique attaché à la partie mobile et à la partie fixe de la broche, l'élément élastique étant de préférence un ressort.

7. Dispositif selon l'une des revendications 1 à 3, dans lequel la partie male (72) est constituée d'une lame (75) ressort s'étendant depuis la face interne (21) du boitier (2) et est conformée pour présenter une partie (751) configurée pour être introduite dans l'orifice (73).

8. Dispositif selon l'une des revendications précédentes, dans lequel la partie femelle (71) comprend un via (713) qui s'étend depuis l'orifice dans l'épaisseur du capteur.

## Patentansprüche

1. Körperüberwachungsvorrichtung, umfassend:
- ein Gehäuse (2), das eine untere Fläche (21) und ein Armband (23) umfasst, das geeignet ist, das Handgelenk eines Benutzers zu umgeben,
- einen Sensor (3), der eine untere Fläche (31), eine obere Fläche (32) gegenüber der unteren Fläche (21) des Gehäuses (2) und mindestens eine Mikronadel (5) umfasst, die auf der unteren Fläche (31) des Sensors (3) angeordnet ist,
- mindestens einen Steckverbinder (7), der das Gehäuse (2) elektrisch mit dem Sensor (3) verbindet, wobei jeder Steckverbinder (7) eine Buchse (71) umfasst, wobei die Buchse (71) eine konkave, in der Dicke des Sensors (3) ausgebildete Aufnahmeöffnung (73) umfasst, sowie einen Stecker (72), der von der unteren Fläche (31) des Gehäuses (2) vorsteht, wobei die Öffnung (73) bezüglich der oberen Fläche (32) des Sensors (3) konkav ist, von der aus die Öffnung (73) gebildet ist,
- einen elektrischen Steckverbinder (33), der auf der unteren Fläche (31) des Sensors (3) angeordnet ist, wobei eine Mikronadel mit dem elektrischen Steckverbinder (33) verbunden ist, wobei die Buchse (71) dazu ausgestaltet ist, so mit dem elektrischen Steckverbinder (33) verbunden zu werden, dass die obere und die untere Fläche des Sensors (3) elektrisch verbunden werden,
wobei die Öffnung (73) derart ist, dass, wenn der Stecker (72) in die Öffnung (73) eingeführt ist, die Kontaktfläche zwischen dem Stecker und der Öffnung größer ist als bei einer ebenen Kontaktierung,
wobei die Buchse (71) durch ein metallisches Teil (712) gebildet ist, das über die gesamte Dicke des Sensors angeordnet ist, wobei das metallische Teil einen konkaven Abschnitt aufweist, von dem sich eine elektrisch leitende metallische Stütze zu der unteren Fläche (31) des Sensors (3) erstreckt,
wobei der Sensor (3) eine Dicke zwischen einschließlich 1 mm und einschließlich 3 mm aufweist.

2. Vorrichtung nach Anspruch 1, wobei die Öffnung (73) kugelförmig oder konisch oder kegelstumpfförmig ist.

3. Vorrichtung nach einem der Ansprüche 1 bis 2, wobei die Öffnung (73) einen Öffnungswinkel zwischen 100°und 160°, vorzugsweise von 130°, aufweist.

4. Vorrichtung nach einem der Ansprüche 1 bis 3, wobei der Stecker ein Kontaktstift (74) ist, der von der unteren Fläche (31) des Gehäuses vorsteht.

5. Vorrichtung nach Anspruch 4, wobei der Kontaktstift einen an dem Gehäuse (2) angebrachten feststehenden Teil und einen bezüglich des feststehenden Teils beweglichen Teil umfasst, wobei der Kontaktstift so ausgestaltet ist, dass er eine Verschiebung des beweglichen Teils bezüglich des feststehenden Teils in einer senkrecht zu der oberen Fläche (32) des Sensors (3) verlaufenden Translationsrichtung (Z) gestattet.

6. Vorrichtung nach Anspruch 5, wobei der Kontaktstift (74) ein elastisches Element umfasst, das an dem beweglichen Teil und an dem feststehenden Teil des Kontaktstifts angebracht ist, wobei das elastische Element vorzugsweise eine Feder ist.

7. Vorrichtung nach einem der Ansprüche 1 bis 3, wobei der Stecker (72) aus einer Blattfeder (75) besteht, die sich von der inneren Fläche (21) des Gehäuses (2) erstreckt und dazu ausgebildet ist, einen Teil (751) darzustellen, der für die Einführung in die Öffnung (73) ausgestaltet ist.

8. Vorrichtung nach einem der vorhergehenden Ansprüche, wobei die Buchse (71) ein Via (713) umfasst, das sich von der Öffnung in die Dicke des Sensors erstreckt.

## Claims

1. Body monitoring device, comprising
- a housing (2) comprising a lower face (21) and a strap (23) designed to encircle the wrist of a user;
- a sensor (3) comprising a lower face (31), an upper face (32) arranged opposite the lower face (21) of the housing (2) and at least one microneedle (5) arranged on the lower face (31) of the sensor (3),
- at least one connector (7) electrically connecting the housing (2) to the sensor (3), each connector (7) comprising a female portion (71), the female portion (71) comprising a concave receiving orifice (73) formed through the thickness of the sensor (3) and a male portion (72) projecting from the lower face (31) of the housing (2), the orifice (73) being concave relative to the upper face (32) of the sensor (3) from which the orifice (73) opens,
- an electrical connector (33) arranged on the lower face (31) of the sensor (3), a microneedle being connected to the electrical connector (33), the female portion (71) being configured to be connected to the electrical connector (33) so as to electrically connect the upper and lower faces of the sensor (3),
the orifice (73) being such that when the male portion (72) is inserted into the orifice (73), the contact surface between the male portion and the orifice is greater than that of a planar contact
wherein the female portion (71) consists of a metal portion (712) arranged through the entire thickness of the sensor, the metal portion comprising the concave portion from which a conductive metal support extends toward the lower surface (31) of the sensor (3), wherein the sensor (3) has a thickness of between 1 mm and 3 mm inclusive.

2. Device according to Claim 1, wherein the orifice (73) is spherical, or conical or frustoconical.

3. Device according to either of Claims 1 and 2, wherein the orifice (73) has an opening angle of between 100° and 160°, preferably 130°.

4. Device according to one of Claims 1 to 3, wherein the male portion is a pin (74) projecting from the lower face (31) of the housing.

5. Device according to Claim 4, wherein the pin comprises a fixed portion attached to the housing (2) and a portion movable relative to the fixed portion, the pin being configured to enable the movable portion to move relative to the fixed portion in a translation direction (Z) perpendicular to the upper face (32) of the sensor (3).

6. Device according to Claim 5, wherein the pin (74) comprises a resilient member attached to the movable portion and to the fixed portion of the pin, the resilient member preferably being a spring.

7. Device according to one of Claims 1 to 3, wherein the male portion (72) consists of a spring blade (75) extending from the inner face (21) of the housing (2) and is shaped to present a portion (751) configured to be inserted into the orifice (73).

8. Device according to one of the preceding claims, wherein the female portion (71) comprises a via (713) extending from the orifice into the thickness of the sensor.
